# EUROPEAN PATENT SPECIFICATION

(11) **EP 4 093 340 B1**
(45) Date of publication and mention of the grant of the patent: **14.10.2026**
(21) Application number: 21745069.1
(22) Date of filing: 25.01.2021
(51) Int. Cl.: A61F 2/50, A61F 2/54, A61F 2/60, A61F 2/76, A61F 2/80

(54) **MANUFACTURING SOCKET OF LOWER AND UPPER LIMB PROSTHESES**
HERSTELLUNG EINES SCHAFTES FÜR PROTHESEN DER UNTEREN UND OBEREN GLIEDMASSEN
DOUILLE DE FABRICATION DE PROTHÈSES DE MEMBRES INFÉRIEURS ET SUPÉRIEURS

(30) Priority: 26.01.2020 US 202062965904 P
(43) Date of publication of application: 30.11.2022
(73) Proprietor: Standelone Medical Ltd., 4809164 Rosh HaAyin (IL)
(72) Inventor: RAMOT, Yair, 4992500 Maas (IL); RAMOT, Roi, 4841025 Rosh Haayin (IL)
(74) Representative: Becker Kurig & Partner Patentanwälte mbB
(86) International application number: PCT/IB2021/050548
(87) International publication number: WO 2021/149023

(56) References cited:
- WO-A1-2019/157486
- GB-A- 1 416 453
- GB-A- 2 116 432
- US-A- 2 613 398
- US-A- 4 327 046
- US-A- 5 503 543
- US-A- 5 503 543
- US-A1- 2002 043 738
- US-A1- 2002 043 738
- US-A1- 2015 142 150
- US-A1- 2017 246 013
- US-A1- 2020 022 819
- US-B1- 6 231 616

## Description

### FIELD OF THE INVENTION

The present invention relates generally to methods and systems for manufacturing sockets for lower and upper limb prostheses.

### BACKGROUND OF THE INVENTION

Prostheses technology has advanced tremendously in recent decades, including prostheses that are lighter and stronger (composite materials), smarter (embedded sensors), integrated with the nervous system (myoelectric), and active (with power and motors). However, the methods for producing the "socket" - the part of the prosthesis that fits onto the person's stump and connects to the other components of the prosthesis - remain archaic. Measuring the stump for the socket and fitting the socket to the person are still highly manual (which is costly and slow) and imprecise (20% to 50% of sockets do not fit well the first time, which is costly, frustrating and depressing).

The importance of a properly fit socket cannot be underestimated: the socket is the main part that determines the level of comfort or pain, sores, bruises, strength, stability and performance.

In the prior art, measuring and fitting a person for a prosthesis socket is a manual process. It requires taking into account stump position, size and shape (which is always irregular), shape changes under pressure and movement, bone protuberances, varying tissue hardness and sensitivity, overall body size and weight, expected usage patterns, and many more factors.

The technician feels the stump tissue, optionally places a stocking-like liner over the stump, marks sensitive areas (e.g., those that are particularly susceptible to abrasion and bone protuberances), and wraps plaster-gauze strips around the stump to create a "negative" of the shape. While the plaster dries and solidifies, the technician presses certain points to create indentations that are aligned with potential feasible pressure points (which will help hold the socket to the stump during use).

Once dry, the technician removes the plaster cast, makes any modifications needed and declares the "negative" ready for use as a mold for the "positive". The technician then fills the negative with liquid plaster, which hardens, forming a "positive". The technician may modify the positive by adding or removing material. The technician then creates the socket, by drawing sheets of composite material over the positive, adhering and shaping them manually, and enabling them to set. The socket extremities may be machined to the desired shape and any rough edges may be sanded.

Document GB 2116432 A discloses a method of making an artificial limb by means of a positive impression of an amputated limb stump produced by first surrounding the stump with a hollow flexible jacket containing polystyrene beads in an inner compartment, inflating an outer compartment if necessary to compress the jacket against the stump, and evacuating the inner compartment so that the beads form a rigid mass moulded to the shape of the stump.

Document US 2613398 A is directed to a method for making inner molds.

Document US 4327046 A discloses a rigid, shaped, mass support system and a method for producing same.

Document US 2017/246013 A1 is directed to a system and method for producing a customized prosthetic socket based on a digital representation of a residual limb that is used to form a digital model of a prosthetic socket. Document US5503543 A is directed to a machine for casting a prosthesis comprising vertical supports.

### SUMMARY OF THE INVENTION

The present invention seeks to provide devices and methods for measuring, manufacturing and fitting sockets, which:
(i) provide better-fitting sockets, measuring the stump under operating loads,
(ii) reduce iterations of measurement, manufacture and fitting,
(iii) reduce cost and delivery-time,
(iv) enhance productivity of the prosthesis industry and
(v) work with the incumbent industry, not against it.

The present invention includes a measuring system and a manufacturing system, as is described hereinbelow.

The dynamic measuring system simulates real time forces and loads including dynamic loads and forces acting on the stump. The dynamic measuring system simulates real time walking and standing and other positioning tasks. The dynamic measuring system simulates the deformation of the residual limb under real loading conditions. The dynamic measuring system simulates in real time and conditions the comfort and satisfactory of the patient. It is a simple system designed to capture the optimal shape and fixate it. The system creates a positive shape using expendable material to enable easy scanning of the desired shape. The design may include different pressure releasing structures at desired locations.

The system can collect data from different patients to give technical advice to help the technician reach optimal clinical results.

The system may include a 3D printing system for manufacturing the socket, which can print at low cost and at different printing speeds using different size nozzles in an optimized format. After printing, the material properties enable performing some minor changes and adaptations to the socket shape to achieve an optimal fit, such as by using a local heating process.

### BRIEF DESCRIPTION OF THE DRAWINGS

The present invention will be understood and appreciated more fully from the following detailed description taken in conjunction with the drawings in which:
Fig. 1 is a simplified pictorial illustration of a measuring system in accordance with a non-limiting embodiment of the present invention, including a deformable measuring bag with holding structure and connection to the lower part of the prosthetic limb.
Fig. 2 is an exploded illustration of the measuring system.
Fig. 3 is another illustration of the measuring system.
Fig. 4 is a simplified illustration of an example of a pressure release structure for pressure sensitive areas on the stump.

### DETAILED DESCRIPTION OF EMBODIMENTS

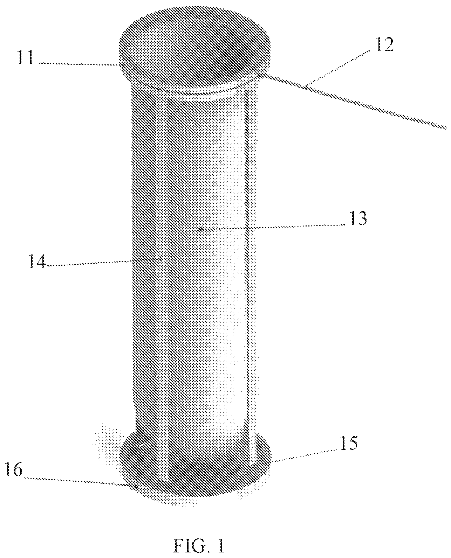

Reference is now made to Fig. 1, which illustrate a measuring system, constructed and operative in accordance with a non-limiting embodiment of the present invention.

The measuring system may include upper connection rings 11, which seal the two parts of a deformable measuring bag 13 and connect it to a measuring jig. An air evacuation tube 12 enables creating a vacuum inside the deformable measuring bag 13. Tube 12 may include a valve for controlling the vacuum level. The deformable measuring bag 13 may be filled with beads or pellets.

Vertical movable supports 14 are provided for supporting the deformable measuring bag 13 during the measuring/simulating process. Adjusting elements 15 are provided for adjusting the vertical supports 14 of the deformable measuring bag 13 during the measurement process.

A lower plate 16 may connect the measuring jig to the lower part of the prosthetic limb to enable full simulation during the measuring process.

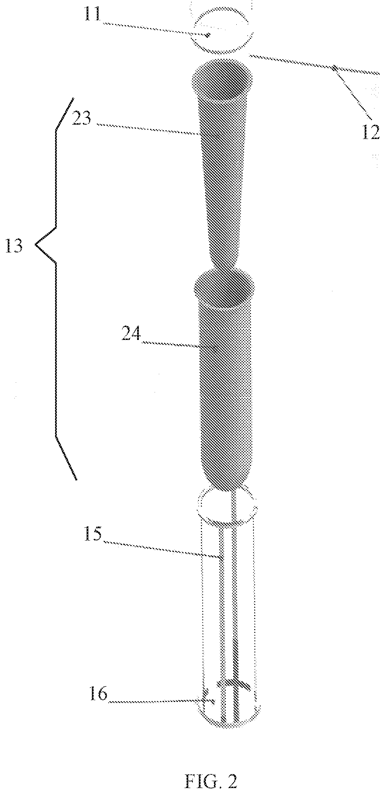

Reference is now made to Fig. 2, which illustrates an exploded view of the system of Fig. 1. The system includes upper connection rings 11, which seal the two parts of the deformable measuring bag 13 and connect it to the measuring jigs. Air evacuation tube 12 is used to create a vacuum inside the deformable measuring bag 13. The deformable measuring bag 13 includes an inner portion 23 and an outer portion 24.

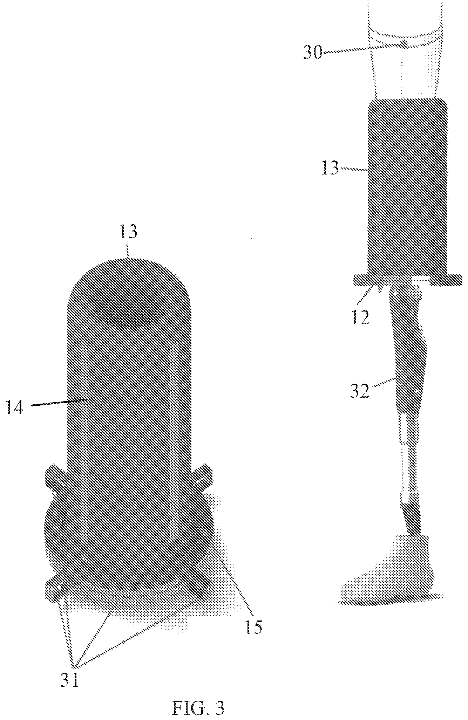

Reference is now made to Fig. 3, which illustrates deformable measuring bag 13 filled with beads or pellets, and which is placed under a residual limb 30 of the patient. The complete supporting structure of the measuring system includes the connecting plate 16 for attachment to a lower leg prosthesis 32, adjustable vertical supports (e.g., rods) and horizontal adjusting supports 31. The connecting plate 16 may include a flat washer for sealing the air inlet to the deformable measuring bag 13.

The invention can measure the shape of the residual limb 30 in a dynamic mode and under real loading conditions with the stump deformed to its standing, walking or working shape. This negates the need for guesswork about how the stump will change shape under stress. This process may be done using the measuring system as a simulator.

The residual limb 30 is inserted into the deformable measuring bag 13 containing small beads, for example, up to 1 mm in diameter, made of material of up to 60 Shore A hardness to capture the 3-dimensional shape of the individual stamp. The deformable measuring bag 13 is pressed from the outside using a circular pressure calf with a preset air pressure to ensure a uniform and controllable pressure at all contact points of the measured socket (i.e., the bag) with the stump. This process sets the pressure to an initial safe level to prevent any pressure related sores or discomfort. At this stage the air is sucked out of the deformable measuring bag 13 by vacuum through tube 12 to a preset vacuum (negative pressure) level in order to set the shape. The patient can apply full load on the measuring system and simulate all relative functions like walking, standing etc. In the second stage the outer pressure calf is removed, and the prosthetic technician can manually create additional pressure points or remove some pressure from different contact points to improve the fitting of the measured socket. This is performed by alternating the air pressure in the deformable measuring bag 13.

Prior to the measuring process the deformable measuring bag 13 is prepared by inserting some two-component resin that will harden the bag within a set time or activated by heat, thereby enabling a preset working time interval.

Once the time is up, the deformable measuring bag 13 hardens to the shape of the stump and creates a durable stable measured socket with the best fit.

The pressure between the stump and the socket can be controlled to achieve safe levels as well by indications from a set of sensors placed on the inner side of the deformable measuring bag 13. This presents to the prosthetic technician a visual pressure map of the contact between the socket and the stump.

Once the final shape of the socket is achieved the shape on the inner cavity created by the deformable measuring bag 13 can be copied to a positive shape using a two component or one component expanding material. This material is poured into the cavity and expands to totally fill the entire space. Once the material hardens it is extracted from the deformable measuring bag 13 and scanned using a 3D scanner to create a 3D file for the next stage of manufacturing the socket.

The expandable material has some elastic properties to simulate the actual stamp and enable to easily extract the created shape out of the deformable measuring bag 13.

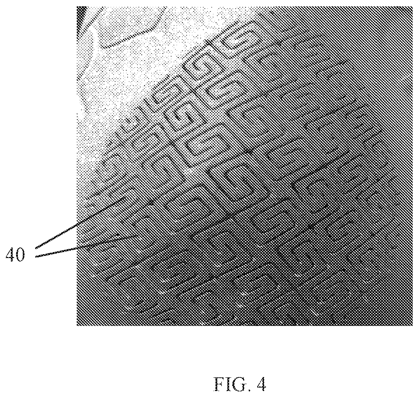

As seen in Fig. 4, the designed socket can incorporate some specially designed shapes 40 to act as pressure release points for pressure sensitive areas on the stump.

The system may include a data collection system from patients to give technical advice to help the technician reach optimal clinical results.

Once this process is completed, one proceeds to the manufacturing stage.

The manufacturing process begins with 3-dimensional scanning of the positive shape created in the previous stage using the expandable material. This 3D file can be presented using 3D design software. The software can enable the prosthetic technician to perform some minor needed changes and adaptations to the designed socket. The system can enable adding the pressure release elements as described above into specific areas on the designed socket.

The software and data base will enable the prosthetic technician to perform the best adaptation to the socket to achieve the optimal fit.

The software will enable the prosthetic technician to add any standard connecting element to the socket for connecting of any kind of a prosthesis.

The complete socket system is then 3D printed on a specially designed 3D printing system. The system is designed to print at low cost and different printing speeds using different size nozzles in an optimized format. The total printing time, without limitation, is less than 360 minutes for a complete socket.

Printing with a large nozzle creates a strong structure with some compromise of accuracy that will not affect the socket performance but will enable a significantly higher printing speed.

## Claims

1. A system for creating a prosthesis comprising:
a deformable measuring bag (13); an air evacuation tube (12) coupled to said deformable measuring bag (13) and connectable to a vacuum source; a connection element (16) for connecting said deformable measuring bag (13) to a prosthetic limb (32) **characterized by** vertical movable supports (14) with adjusting elements (15) for adjusting the vertical supports, the supports being coupled to said deformable measuring bag (13) for supporting said deformable measuring bag (13).

2. A method for creating a prosthesis, the method comprising:
providing a system according to claim 1;
filling the deformable measuring bag (13) with a hardenable material;
connecting a lower portion of the bag (13) to a prosthetic limb (32);
inserting a stump of a residual limb of a patient into an upper portion of the deformable measuring bag (13);
pressing the deformable measuring bag (13) against the stump to ensure a uniform and controllable pressure at all contact points of the deformable measuring bag (13) with the stump;
sucking air out of the deformable measuring bag (13) by vacuum to set a shape of the bag (13), during which the patient can apply different loads while walking or standing;
allowing the hardenable material to harden in the bag (13) and form a negative mold;
pouring a hardenable substance into said negative mold and allowing said hardenable substance to harden to form a positive mold;
extracting said positive mold and scanning said positive mold to create a 3D computer file; and
printing a prosthetic socket using said 3D file.

3. The method according to claim 2, comprising using sensors to sense pressure between the stump and the bag (13).

4. The method according to claim 2, comprising adding pressure release points for pressure sensitive areas on the stump.

## Patentansprüche

1. System zur Herstellung einer Prothese, umfassend:
einen verformbaren Messbeutel (13); einen Luftabsaugschlauch (12), der mit dem verformbaren Messbeutel (13) verbunden ist und mit einer Vakuumquelle verbunden werden kann; ein Verbindungselement (16) zum Verbinden des verformbaren Messbeutels (13) mit einer Prothesengliedmaße (32),
**gekennzeichnet durch** vertikal bewegbare Stützen (14) mit Einstellelementen (15) zum Einstellen der vertikalen Stützen (14), wobei die Stützen mit dem verformbaren Messbeutel (13) gekoppelt sind, um den verformbaren Messbeutel (13) zu stützen.

2. Verfahren zur Herstellung einer Prothese, wobei das Verfahren umfasst:
Bereitstellen eines Systems gemäß Anspruch 1,
Befüllen des verformbaren Messbeutels (13) mit einem aushärtbaren Material;
Verbinden eines unteren Abschnitts des Beutels (13) mit einer Prothesengliedmaße (32);
Einführen eines Stumpfs eines Restglieds eines Patienten in einen oberen Abschnitt des verformbaren Messbeutels (13);
Anpressen des verformbaren Messbeutels (13) an den Stumpf, um einen gleichmäßigen und kontrollierbaren Druck an allen Kontaktpunkten des verformbaren Messbeutels (13) mit dem Stumpf sicherzustellen;
Absaugen von Luft aus dem verformbaren Messbeutel (13) mittels Vakuum, um eine Form des Beutels (13) festzulegen, wobei der Patient beim Gehen oder Stehen unterschiedliche Belastungen ausüben kann;
Erlauben des Aushärtens des aushärtbaren Materials im Beutel (13) und der Bilden einer Negativform;
Gießen einer aushärtbaren Substanz in die Negativform und Aushärtenlassen dieser Substanz, um eine Positivform zu bilden;
Entnehmen der Positivform und Scannen der Positivform, um eine 3D-Computerdatei zu erstellen; und
Drucken eines Prothesenschafts unter Verwendung der 3D-Datei.

3. Verfahren gemäß Anspruch 2, umfassend die Verwendung von Sensoren zum Erfassen des Drucks zwischen dem Stumpf und dem Beutel (13).

4. Verfahren gemäß Anspruch 2, umfassend das Hinzufügen von Druckentlastungspunkten für druckempfindliche Bereiche am Stumpf.

## Revendications

1. Un système destiné à la création d'une prothèse, comprenant :
un sac de mesure déformable (13) ; un tube (12) d'évacuation d'air, relié audit sac de mesure déformable (13) et apte à être raccordé à une source de vide ;
un élément de liaison (16) destiné à relier ledit sac de mesure déformable (13) à un membre prothétique (32), **caractérisé par** des supports mobiles verticaux (14) comprenant des éléments de réglage (15) pour régler les supports verticaux, lesdits supports étant reliés audit sac de mesure déformable (13) afin de soutenir ledit sac de mesure déformable (13).

2. Un procédé de création d'une prothèse, le procédé comprenant :
le fait de se mettre à disposition un système selon la revendication 1 ;
le fait de remplir le sac de mesure déformable (13) avec un matériau durcissable ;
le fait de relier une partie inférieure du sac (13) à un membre prothétique (32) ;
le fait d'insérer le moignon d'un membre résiduel d'un patient dans une partie supérieure du sac de mesure déformable (13) ;
le fait d'appuyer le sac de mesure déformable (13) contre le moignon afin d'assurer une pression uniforme et contrôlable en tous les points de contact du sac de mesure déformable (13) avec le moignon ;
le fait d'aspirer l'air hors de la poche de mesure déformable (13) par le vide afin de fixer la forme du sac (13), ce pendant lequel le patient peut exercer différentes charges en marchant ou en se tenant en position debout ;
le fait de laisser le matériau durcissable durcir dans le sac (13) et former un moule négatif ;
le fait de verser une substance durcissable dans ledit moule négatif et de laisser ladite substance durcissable durcir pour former un moule positif ;
le fait d'extraire ledit moule positif et le numériser pour créer un fichier informatique en 3D ; et
le fait d'imprimer un manchon prothétique au moyen dudit fichier 3D.

3. Le procédé selon la revendication 2, comprenant le fait d'utiliser des capteurs pour détecter la pression entre le moignon et le sac (13).

4. Le procédé selon la revendication 2, comprenant le fait d'ajouter des points de relâchement de pression pour les zones sensibles à la pression sur le moignon.
